# EUROPEAN PATENT APPLICATION

(11) **EP 0 987 025 A1**
(43) Date of publication of application: **22.03.2000**
(21) Application number: 98830479.6
(22) Date of filing: 04.08.1998
(51) Int. Cl.: A61K 31/70

(54) **Pharmaceitical composition or composition package containing a pyrimidine nucleoside analogue and a purine nucleoside analogue**

(71) Applicant: Magnani, Mauro, 61029 Urbino (IT); Brandi, Giorgio, 61033 Fermignano PS (IT); Fraternale, Alessandra, 61029 Urbino (IT); Casabianca, Anna, 61025 Montelabbate PS (IT)
(72) Inventor: Magnani, Mauro, 61029 Urbino (IT); Brandi, Giorgio, 61033 Fermignano (PS) (IT); Fraternale, Alessandra, 61029 Urbino (IT); Casabianca, Anna, 61025 Montelabbate (PS) (IT)
(74) Representative: Forattini, Amelia

(57) **Abstract**

The invention relates to a pharmaceutical composition or composition package containing a nucleoside analogue (a) selected from the group consisting of reverse transcriptase inhibitors being pyrimidine nucleoside analogues. The invention is characterized in that it further contains a nucleoside analogue (b) selected from the group consisting of arabinonucleosides and halogenated and/or 5*'* phosphorylated and/or 2*'*and/or 3*'*desoxy- derivatives thereof.

## Description

The invention relates to a pharmaceutical composition or composition package containing a nucleoside analogue (a) selected from the group consisting of reverse transcriptase inhibitors being pyrimidine nucleoside analogues, to a use of nucleoside analogues for manufacturing a pharmaceutical composition or composition package for treating diseases caused by lentivirus infection, e.g. AIDS, to a process for making such a pharmaceutical composition or composition package, and to a method for treating said diseases. - A pharmaceutical composition as defined herewith contains more than one, preferably two, pharmaceutically active compounds mixed with each other. A pharmaceutical composition package as defined herewith comprises at least two, preferably two, package components, each of which contains at least one, preferably one, pharmaceutically active compound. The different package components are determined to be administered separately. The separate administration may be by different methods of administration, e.g. oral administration or injection (i.p., i.v., etc.). The separate administration may further and independent from the method of administration be performed at the same time or subsequently. Subsequent administration may be according to a therapy plan comprising the administration of one package component for a certain period of time and the administration of the second package component for a subsequent certain period of time, and so on. During subsequent administration of the second, third, and so on, package component, the administration of the first, second, and so on package component may be continued or interrupted. Further, the administration routine as described may be repeated in repeating cycles, whereby the administration routine in each cycle may differ with respect to the duration of administration and/or doses of administration. Nucleoside analogues are synthetic compounds chemically and physiologically related with the naturally occurring nucleosides, including the desoxynucleosides. Such nucleoside analogues interact within the natural metabolism (including transcription) of cells, bacteria or virus and result in the natural metabolism being disturbed or interrupted. This may, in particular, be utilized for the treatment of diseases due to viral infections because the metabolism of the virus and/or the metabolism of a cell infected by the virus may selectively be disturbed with the result of virus action inhibition. Reverse transcriptase inhibitors are compounds which inhibit totally or partially the enzyme catalyzing the transcription from RNA to DNA, as occurring e.g. in retroviral interaction with infected cells. Lentivirus is a group of retrovirus including HIV, SIV, FIV, BIV, and RNA-Onkovirus. HIV causes the disease AIDS in human beings.

The use of 3*'*-Azido-3*'*-desoxythymidin for the treatment of AIDS is well known. 3*'*-Azido-3*'*-desoxythymidin is a pyrimidine nucleoside analogue acting as a reverse transcriptase inhibitor and is also named Zidovudin and AZT. It is further well known that current HAART therapy, in which two nucleoside analogues (usually AZT and 3TC) and a protease inhibitor are used, reduces viral load in the blood of HIV-1 infected patients by 99% in the first two weeks of treatment. Unfortunately, this exponential virus decline of nearly 2-log is followed by a much slower exponential decline (Perelson et al. Nature 1997, 387: 188-191). Furthermore, the infection progresses even though only a small fraction of infected cells is present (Wain-Hobson Nature 1997, 387: 123-124).

Thus, the technical problem underlying the invention is to provide an antiviral approach with which the eradication of lentivirus, in particular HIV, from the long-lived compartments becomes possible.

This technical problem is solved according to the invention by a pharmaceutical composition or composition package containing a nucleoside analogue (a) selected from the group consisting of reverse transcriptase inhibitors being pyrimidine nucleoside analogues, characterized in that it further contains a nucleoside analogue (b) selected from the group consisting of arabinonucleosides and halogenated and/or 5*'* phosphorylated and/or 2*'*and/or 3*'*desoxy- derivatives thereof. - Arabinonucleosides are purine- or pyrimidinebased nucleosides in which the ribose group has been replaced by the arabinofuranosyl group. Halogenation may be applied to the purine or pyrimidine group, either by substitution of H, or amine group or by addition to >C=O. Phosphorylation at 5*'* may be in form of the monophosphate, the diphosphate or triphosphate. In case of 2*'* and/or 3*'* desoxy- derivatives, the 2*'* or 3*'* OH group is substituted by H or by a functional group like amine, azide and/or halogen. Besides the nucleoside analogues (a) and (b), further nucleoside analogues (c) different from the nucleoside analogues (a) and (b) may be present. The nucleoside analogue (c) may itself be a mixture of several nucleoside analogues. Additionally, one or several protease inhibitors may be present. In the embodiment of a pharmaceutical composition package, the nuoleoside analogue (a) is one main component of a package component (A) and nucleoside analogue (b) is the main component of a package component (B). The package component (A) may consist itself of package subcomponents, each of which contains at least one compound of the group consisting of the nucleoside analogues (a) and (c) and protease inhibitors. In particular, the package component (A) may be constituted like the usual pharmaceutical compounds or compound packages used in the HAART therapy of AIDS. HAART is the abbreviation for Highly Active Antiretroviral Therapies (J. Laurence. HAART Regiments: Do the effects last? The Aids Reader 7(6): 84-85, 1997 As an example, nucleoside analogues AZT and 3TC being inhibitors of the reverse transcriptase are used together with a protease inhibitor. Nucleoside analogues (a) and (b) are easily synthesized by a person skilled in the art as far as not freely available anyway.

The invention is based on the finding that using a nucleoside analogue (b) in combination with a nucleoside analogue (a) can be part of a new therapeutic approach by which a considerable synergistic effect is obtained. In fact, the administration of the nucleoside analogue (b) to patients already on antiviral therapy based on the nucleoside analogue (a), in particular HAART therapy, can contribute in the elimination of the virus from those cell compartments already infected and not protected by the triple drug combination therapy (the nucleoside analogues AZT and 3TC being inhibitors of the reverse transcriptase protecting the cells from new rounds of infection, while the protease inhibitors block the production of new viral particles from cells already infected). In other words, HAART therapy will contribute to reduce the production of new viral particles from infected cells and to protect uninfected cells. The nucleoside analogue (b) will then eliminate the infected cells and the virus therein (and maybe also a number of uninfected lymphocytes). Thus, the nucleoside analogue (b) is preferably administered for comparatively short periods of time (3-4 weeks). This drug treatment may be repeated two-three times until no proviral-containing cell is present.

The functional background of the invention may be understood on the basis of the data presented in the Examples of this specification. These results clearly show that the administration of a nucleoside analogue (b) to LP-BM5 infected mice reduces the progression of the disease and the viral load in the spleen and lymph nodes. This effect is interesting and was further investigated in combination with classical antiviral therapy (i.e. the administration of a nucleoside analogue (a) that is an inhibitor of the reverse transcriptase; AZT). All the parameters investigated support the conclusion that the (preferably sequential) administration of the two drugs is highly effective with additive antiviral effects. The particular role of the nucleoside analogue (b) in the pathogenesis of AIDS is not clear, however few observations can help in drawing a rationale. It was observed that the nucleoside analogue (b) reduces the proviral DNA content in lymph nodes of infected animals. This reduction could be the result of a direct inhibition by the triphosphate of the nucleoside analogue (b) of viral reverse transcriptase or can be the result of the induced apoptosis of virus producing cells (thus reducing viral spread in the infected animals), or both. To distinguish among these different mechanisms a triphosphate of a nucleoside analogue (b) was tested as an in vitro inhibitor of Molony murine leukemia virus reverse transcriptase. The triphosphate was synthesized and then a new reverse transcriptase assay to test the triphosphate was developed. With these tools it was possible to obtain direct evidence that the triphosphate can be considered a true reverse transcriptase inhibitor, acting as chain terminator. On the other hand some of the nucleoside analogues (b) are well known for their ability to induce apoptosis and a significant reduction of B circulating lymphocytes was observed in all the experiments performed. Thus, the most likely conclusion is that the nucleoside analogue (b) in vivo acts by both mechanisms i.e. by inhibition of reverse transcriptase and killing of infected cells. It is worth noting that the nucleoside analogue (b) under the conditions used does not cause any relevant sign of toxicity as evaluated by histological examination of liver and lymph node sections and by measurements of mitochondrial DNA content. Furthermore, the low number of circulating B cells after nucleoside analogue (b) administration returns to normal values after Fludara suspension. The animal data obtained support this view. Furthermore, preliminary data on in vitro HIV-1 infected macrophages suggest that the nucleoside analogue (b) at very low concentrations (0.01 µg/ml) can inhibit viral production and that at slightly higher concentrations can eliminate infected cells. This in vitro behavior of the nucleoside analogue (b) is not similar to any other antiblastic agent. In conclusion, the animal data obtained suggest that the nucleoside analogue (b) should not be considered another nucleoside analogue with antiviral activity but a drug with a potential place in antiviral therapy. Conventional antiviral treatments followed by nucleoside analogue (b) administration for short periods of time should reduce viral infection to a "minimal residual disease" that with 3-4 cycles every two-three months can likely eradicate the virus.

In a preferred embodiment of the invention, the nucleoside analogue (a) is 3*'*-Azido-3*'*-desoxythymidin. AZT is freely available. It is further preferred that the nucleoside analogue (b) is an, optionally 2*'* and/or 3*'* desoxy-, adeninarabinoside halogenated at position 2 and/or phosphorylated at position 5*'*. Of particular advantage is a nucleoside analogue (b) being 2-X-9-β-D-Arabinofuranosyl-9H-purin-6-amine or the 5*'* mono-, di-, or triphosphate thereof, whereby X is F, Cl or Br, preferably F. In case of the X being F this compound is also known as Fludara (5*'* monophosphate) or Fludarabine. Fludarabine or the 5*'* monophosphate thereof are freely available. 5*'* Di- or triphosphates are easily synthesized by a person skilled in the art.

The invention is further directed to the use of a nucleoside analogue (a) selected from the group consisting of reverse transcriptase inhibitors being pyrimidine nucleoside analogues and of a nucleoside analogue (b) selected from the group consisting of arabinonucleosides and halogenated and/or phosphorylated and/or 2*'* and/or 3*'* desoxy- derivatives thereof for manufacturing a pharmaceutical composition or composition package for treating diseases caused by lentivirus infection, in particular for treating AIDS. Preferably, nucleoside analogues (a) and (b) according to the claims 6 to 8 are used.

The invention further relates to the use according to one of the claims 5 to 8 in the embodiment of a pharmaceutical composition package comprising a package component (A) containing the nucleoside analogue (a) and a package component (B) containing the nucleoside analogue (b), whereby the package components (A) and (B) are determined to be administered according to a therapy plan comprising sequential administration of the package components (A) and (B). Preferably, the package component (A) is determined for administration prior to the administration of the package component (B). It is advantageous if the package component (A) is determined for administration for a period of time of 2 - 20 weeks, preferably 4 to 8 weeks, and the package component (B) is determined for administration for a subsequent period of time of 2 - 20 weeks, preferably 3 - 6 weeks, whereby the administration of the of the package component (A) is either maintained or interrupted during the administration of the package component (B). It is helpful to administer the package components (A) and (B) in repeating cycles, e.g. 2 - 10, preferably 3 - 4, cycles.

The invention is also directed to a process for making a pharmaceutical composition or a pharmaceutical composition package, characterized in that a nucleoside analogue (a) selected from the group consisting of reverse transcriptase inhibitors being pyrimidine nucleoside analogues and a nucleoside analogue (b) selected from the group consisting of arabinonucleosides and halogenated and/or phosphorylated and/or 2*'* and/or 3*'* desoxy- derivatives thereof are used, whereby optionally usual galenic additives are admixed to the mixture of the nucleoside analogues (a) and (b) or are separately admixed to package components (A) (or package subcomponents, e.g. according to the HAART therapy, thereof) and (B), each containing one of the nucleoside analogues (a) and (b), respectively. Preferred embodiments of the process according to the invention are defined in the claims 14 to 16.

Finally, the invention is directed to a method for treating diseases caused by lentivirus infections, in particular for treating AIDS, whereby a pharmaceutical composition or composition package according to one of the claims 1 to 4 is administered to the infected organism. In a preferred embodiment a composition package comprising a package component (A) (optionally consisting of package subcomponents according to the HAART therapy) containing the nucleoside analogue (a) and a package component (B) containing the nucleoside analogue (b) are used, and the package components (A) and (B) are administered according to a therapy plan consisting of sequential administration of the package components (A) and (B). In detail, the package component (A) may be administered prior to the administration of the package component (B). The package component (A) may be administered for a period of time of 2 - 20 weeks, preferably 4 - 8 weeks, and the package component (B) is administered for a subsequent period of time of 2 - 20 weeks, preferably 3 - 6 weeks, whereby the administration of the package component (A) is either maintained or interrupted during the administration of the package component (B). The package components (A) and (B) may administered in repeating cycles, e.g. 2 to 10 cycles, preferably 3 - 4 cycles.

In case of the subject matters of the claims 5 to 21, the explanations and definitions given for the subject matters of the claims 1 to 4 apply in the same sense. With respect to the dose of the nucleoside analogue (a) when treating human beings reference is made to the HAART therapy and the dose values applied therein. The dose of the nucleoside analogue (b) when treating human beings should be in the range from 10 mg/m²/day to 90 mg/m²/day, preferably 40 mg/m²/day.

The total dose of the nucleoside analogue should be in the range from 50 mg/m² to 450 mg/m², preferably 200 mg/m².

This total dose definition applies to one cycle if several administration cycles are performed.

Following the invention is explained in detail by non-limiting experimental examples.

### Experimental details

LP-BM5 virus was provided by Robert Yetter (Veternas Administration Hospital, Baltimore, MD) and was maintained in a persistently infected SC- 1 cell line as described (Hosier D.E., Yetter R.A., Morse H.C. III, "Functional T lymphocytes are required for a murine retrovirus induced immunodeficiency disease (MAIDS)", J. Exp. Med. 1987; 165:1737-42). C57BL/6 female mice were purchased from Nossan, Milan, Italy and were used at 5 weeks of age. Animals were infected by means of two successive intraperitoneal injections at 24-hr intervals. In the experiments reported three different virus preparations were used.

Mice were housed at 22±1°C with a 12-hr light-dark cycle, 60±5% humidity, and 12 air changes/hr.

2-Fluoro-ara (F-ara-AMP, Fludara) was provided by Schering S.p.A., Milan.

AZT (Sigma) was administered ad libitum in the drinking water at a concentration of 0.25 mg/ml. AZT was stable in the drinking water at room temperature and was changed every 3 days.

### Serum immunoglobulin determination

Serum IgG levels were determined using an ELISA technique. Briefly, polyvinylchloride microtiter plates (Immulon I; Dynatech Laboratories, Chantilly, VA) were coated with goat anti mouse IgG (Sigma) diluted in 0.135 M sodium chloride for ≥ 24 hr. After four washes with 0.1% tween PBS (TPBS), the plates were blocked with 5% bovin serum albumin (BSA) in PBS for 1 h at 37°C and washed again with TPBS. Serial dilutions of mouse serum were added and after incubation for 1 h at 37°C, the plates were washed four times with TPBS, and 100 µl of horseradish (HRP)-conjugated goat anti-mouse IgG (Bio-Rad, Richmond, CA) was added. After 1 h incubation at 37°C, serum levels were determined using a color development solution containing 2.2 mM O-Phenylenediamine. Absorbance was measured at 492 nm on Model 2550 EIA READER (BIO-RAD). Absolute serum IgG concentrations were obtained using known concentrations of standard mouse IgG.

### Flow citometry analysis

Whole blood samples were stained with antimouse L3T4/CD₄ fluorescein isothiocyanate (FITC) conjugated (Boehringer Mannheim Biochemica) for the detection of CD4⁺ lymphocytes, antimouse Ly-2R phycoerythrin (PE) conjugated (Boehringer Mannheim Biochemica) for the detection of CD8a lymphocytes, antimouse Thy-1.2 FITC conjugated (Boehringer Mannheim Biochemica) for the detection of T cells, and antimouse Ly 5 (B220)-PE Monoclonal antibody (Cederlane, Ontario, Canada) for the detection of committed B cell precursors. Samples were analyzed by FACS (Becton Dickinson).

### Histopathological examinations

Samples of lymph nodes and liver from three animals in each treatment group were fixed in 4% paraformaldehyde in phosphate-buffered saline, sectioned, and stained with May Grunwald-Giemsa.

### Assay for DNA synthesis

Spleen lymphocytes were obtained from all experimental groups and distributed in quadruplicate in multiwell (96) plates at 5 x 10⁵ cells/well in a volume of 200 µl. DNA synthesis was assayed after 3 days of culture by measuring [³H]thymidine incorporation in lymphocytes stimulated with 10 µg/ml or phytohemagglutinin (PHA) for T cells and 50 µg/ml licopolysacoharide (LPS) for B cells.

### Primers and probe

The following oligonucleotide primers were used for the amplification of a 141-bp *gag* gene of defective virus genome (BM5d); 5' primer, 5'-AACCTTCCTCCTCTGCCA-3' (sense) corresponding to nucleotides 1456-1473 and 3' primer, 5'-ACCACCTCTCGGGCTTTC-3' (antisense) corresponding to nucleotides 1579-1596 of the BM5d genome (Chat topadhyay SK, Sengupta D.N., FredricksonT.N., Morse H.C. III, Hartley J.W., "Characteristics and contributions of defective, ecotropic and mink cell focus-inducing viruses in a retrovirus induced immunodeficiency syndrome of mice.", J..Virol. 1991; 65: 4232-41.). A second pair or oligonucleotide primers was used for amplification of 203-bp of the mouse glucose-6-phosphate dehydrogenase (G6PD) gene. This amplification served as an internal control (endogenous standard) for evaluation of the relative BM5d proviral DNA. The nucleotide primers for the amplification were as follows: 5' primer, 5'-TGTTCTTCAACCCCGAGGAGT-3' (sense) and 3' primer, 5'-AAGACGTCCAGGATGAGGTGATC-3' (antisense) (Kurdi-Haidar B., Mason P.J., Berrebi A., et al., "Origin and spread of the glucose-6-phosphate dehydrogenase variant (G6PD-Mediterranean) in the Middle East.", Am. J. Hum. Genet. 1990; 47:1013-19.).

Probe D30, which was derived from the 137-bp *Smal-Xhol* p12 gag fragment of the defective virus genome (DU5H), was kindly provided by P. Jolicoeur (Montreal, Canada) (Aziz D.C., Hanna Z., Jolicoeur P., "Severe immunodeficiency disease induced by a defective murine leukemia virus.", Nature 1989; 338: 505-08).

The probe glucose-6-phosphate dehydrogenase (G6PD) represented the 203-bp of the murine genome G6PD gene probe was obtained in our laboratory by PCR using the following oligonucleotide primers: 5' primer, 5'-TGTTCTTCAACCCCGAGGACT-3' (sense) and 3' primer, 5'-AAGACGTCCAGGATGAGGTGATC-3' (anti sense).

To detect the mitochondrial DNA a 1544-bp mitochondrial DNA fragment was used as a probe. The mitochondrial DNA probe (kindly provided by Dr. Gadaleta, University of Bari, Italy) spans the region from nucleotide 2578 to 4122 in the complete human mitochondrial DNA. As a result of the high homology between human and mouse mitochondrial DNA this probe recognizes human and mouse sequences equally well (data not shown) (Bibb M.J., Van Etten R.A., Wright C.T., Walberg M.W., Clayton D.A., "Sequence and gene organization of mouse mitochondrial DNA.", Cell 1981; 26:167-80).

### Preparation of cellular DNA

Total cellular DNA was isolated from the spleens, lymph nodes and bone marrow of uninfected, infected and infected/treated mice by the sodium iodide isopropanol method.

### PCR analyses of LP-BM5 proviral DNA

PCR amplifications were performed in a 25 µl final volume using the Perkin Elmer GeneAMP kit (1 mM MgCl₂, 2.5 U AmpliTag) with 0.3 µg total cellular DNA and 20 pmol of each primer.

PCR reaction were carried out for 37 cycles at 95°C for 30 sec, 58°C for 30 sec and 72°C for 30 sec with a final extension at 72°C for 10 min.

Products were analyzed by electrophoresis on agarose gel, blotted and hybridized with either the ³²P-labeled D30 or G6PD probe.

### Determination of mitochondrial DNA content

Total cellular DNA (4 µg) was digested with the restriction enzyme BamHI (Boehringer, Milan, Italy), analyzed on 0.8% agarose gel, blotted and hybridized with the 1544-bp mitochondrial probe (Brandi G., Casabianca A., Schiavano G.F., et al., "Efficacy and toxicity of long-term administration of 2',3'-dideoxycytidine in the LP-BM5 murine induced immunodeficiency model.", Antiviral Chem. Chemother. 1995; 6:153-61.).

Labeling of the DNA probes was achieved using the random primer DNA-labeling kit from Bio-Rad (Hercules, CA, USA). All reaction protocols were performed according to the manufacturer's instructions.

### Other determinations

The intensity of radiolabeled bands was analyzed and quantified in a GS-250 Molecular Imager™ (Bio-Rad, Hercules, CA, USA). For BM5d proviral DNA detection the relative integration was determined by dividing the intensity of the BM5d bands by the intensity of G6PD bands (Fraternale A., Casabianca A., Rossi L., et al., "Inhibition of murine AIDS by combination of AZT and dideoxycytidine 5'-triphosphate.", J. Acquir. Imm. Def Syndr. and Hum. Retrovir. 1996; 12:164-73). The percentages of inhibition were referred to the infected mice.

The relative amount of mitochondrial DNA in infected/treated mice was compared to the uninfected and infected/not treated mice.

Statistical analysis of data was performed with the parametric test (T-test) of Statquik program (Lundon Software Inc., OH, USA).

### RESULTS

### Effect of Fludara on murine AIDS

First, the efficacy of 2-fluoro-ara-AMP (F-araAMP, Fludara) in inhibiting murine AIDS (MAIDS), a disease characterized by a lymphoproliferative disorder (lymphadenopathy and splenomegaly), associated with severe immunodeficiency and hypergammaglobulinemia, was evaluated. The disease develops in some strains of mice (C57BL/6) that are infected with a murine leukemia virus mixture termed LP-BM5. In the first preliminary experiments (after about 4 months from infection) LP-BMS infected animals were treated with three different concentrations of F-ara-AMP: 0.065 mg/mouse, 0.325 mg/mouse, 0.650 mg/mouse. The animals of each group were treated intraperitoneally for 3 days a week and for 3 weeks. At the end of the treatment animals were killed and splenomegaly, lymphadenopathy and hypergammaglobulinemia were evaluated. The percentages of inhibition of splenomegaly lymphadenopathy and hypergammaglobulinemia are reported in Table I. The results obtained suggested that F-ara-AMP was effective in reducing the signs of the disease and that its effect was dose-depending.

### Identification of the best treatment regime

The following experiments were aimed at defining the most effective dose of drug able to inhibit MAIDS. Infected mice after 7 weeks from infection were treated intraperitoneally with Fludara as follows:
1. 0.65 mg/mouse once a day for 4 days a week (total dose 7.8 mg/mouse);
2. 0.65 mg/mouse twice a day for 5 days a week (total dose 19.5 mg/mouse);
3. 3.00 mg/mouse once a day for 5 days a week (total dose 45 mg/mouse). All treatments lasted 3 weeks.

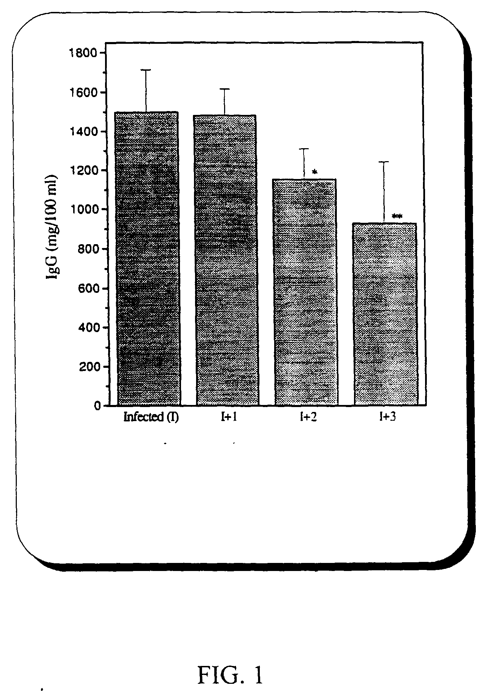

At the end of the treatments mice were sacrificed and splenomegaly, lymphadenopathy and hypergammaglobulinemia were evaluated. The results obtained, reported in the Table II and Fig. 1 confirmed the results already obtained and showed that the most effective dose was 3.00 mg/mouse. In Fig. 1 the numbers 1, 2 and 3 stand for the respective treatment conditions in Table II, ∗ = p<5·10⁻², ∗∗ = p<5·10⁻³[vs. infected].
In the following experiment the efficacy and toxicity or intraperitoneal and oral administration of F-ara-AMP was evaluated. Four groups of infected mice were treated with F-ara-AMP as reported in Table III.

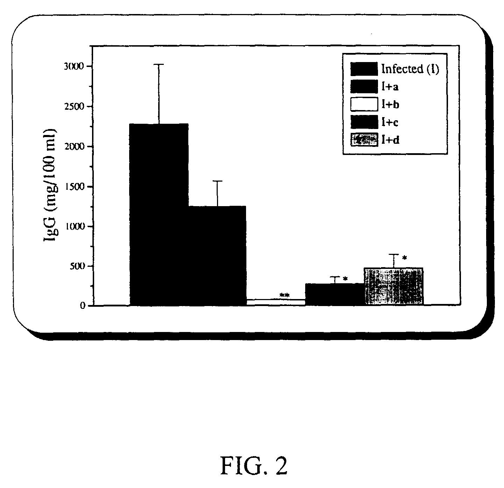

At the 12th week all animals were killed and the results obtained are reported in Tables IV to VI and Fig. 2. The details of the treatments a - d in Fig. 2 are given in the tables IV - VI, ∗∗ = p<10⁻⁴, ∗ = p<5·10³[vs. infected].

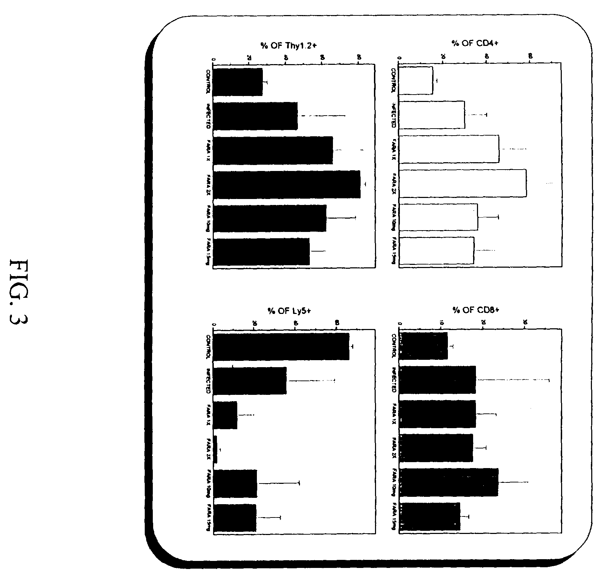

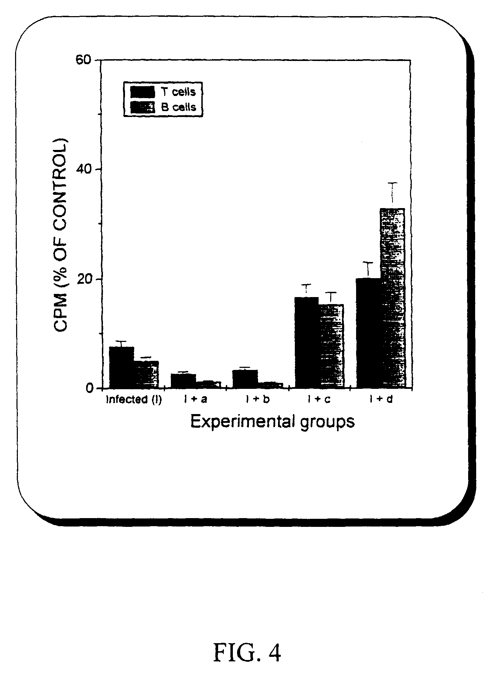

It is worth noting that treatment b (i.e. intraperitoneal administration of Fludara at 3 mg/mouse, twice a day for four weeks) provided the best results in terms of reduction of splenomegaly, lymphadenopathy, hypergammaglobulinemia and reduction of proviral DNA content in spleen and lymph nodes. This last parameter is particularly important since it provides direct evidence that Fludara does not only reduce the progression of the disease but has a true antiviral effect reducing the viral load. As expected Fludara strongly reduces circulating B cells (Fig. 3) and as a consequence the percentage of T cells increases. It is believed that this data should be evaluated with caution since the increase in percentage of T cells is likely a consequence of the depletion of B cells from the total circulating lymphocytes. Spleen lymphocyte response to mitogenic stimuli is not improved by Fludara administered intraperitoneally but it is slightly improved by the oral administration (Fig. 4). Histopathological examination of liver and lymph nodes sections confirmed that Fludara was able to reduce lymphoid infiltrates. The drug administered in drinking water at 15 mg/mouse causes some toxicity as evidenced by the presence of necrotic cells in the liver.

### Effect of Fludara and AZT on the development of murine AIDS

Having established that Fludara administered intraperitoneally at 3 mg twice a day for four weeks is beneficial in LP-BM5 infected mice the administration of Fludara in combination with a typical antiretroviral nucleoside analogue, AZT, was evaluated.

In a first experiment several groups of infected mice were treated with AZT (in drinking water), Fludara, AZT first and then Fludara AZT and Fludara together as reported in Table VII.

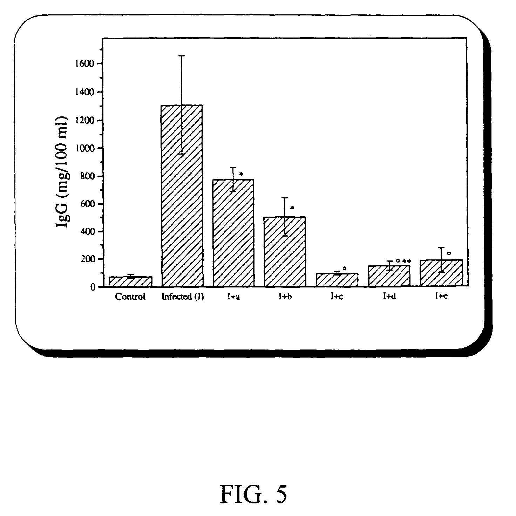

It is immediately evident that the sequential administration of AZT and Fludara provides very interesting results. Based on the evaluation of splenomegaly, lymphadenopathy and hypergammaglobulinemia (Fig. 5; °=p<10⁻³, *=p<10⁻² [vs. Infected]; **=p<5x10⁻² [vs. I+b]) these animals are indistinguishable from normal controls. The hematological parameters in these animals are shown in Table VIII.

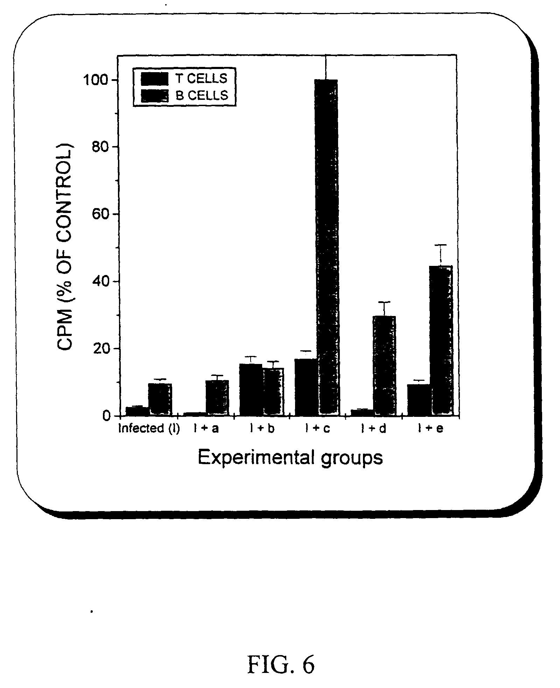

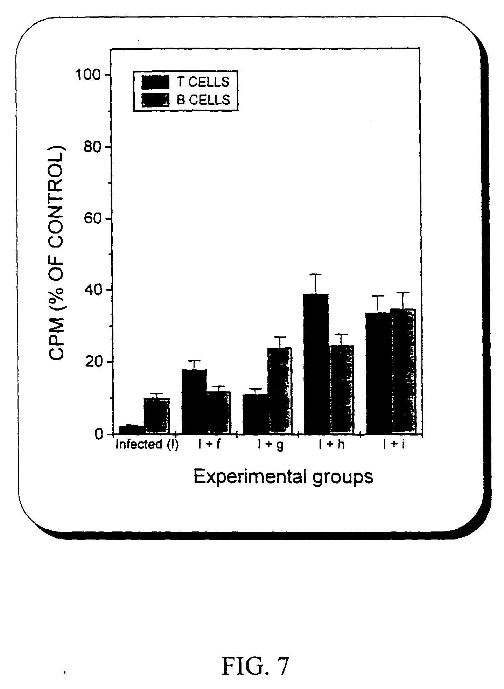

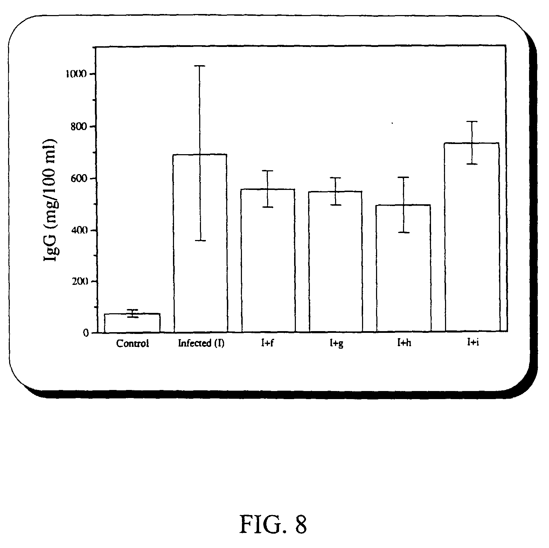

Due to the good results reported above it was decided to carefully evaluate the proviral DNA content in these animals by a quantitative PCR assay. The results obtained (Table IX) provide direct evidence that the sequential administration of AZT and Fludara is better than AZT or Fludara alone and provides a reduction of BM5d proviral DNA of 96.88% in lymph nodes, 94.15% in spleen and 93.45% in the bone marrow. Thus, this antiviral treatment provides about a 2 log reduction of viral load in MAIDS, a value that in HIV- 1 infected patients is obtained by the combination of nucleoside analogues and protease inhibitors (HAART therapy). Administration of Fludara and AZT partially restores the spleen lymphocyte response to mitogenic stimuli (Fig. 6).

Histopathological examination of liver and lymph node sections further supports the results described above, i.e. the ability of sequential treatment with AZT and Fludara in normalizing architecture and cellularity in lymph nodes and complete disappearance of lymphoid infiltrates in liver.

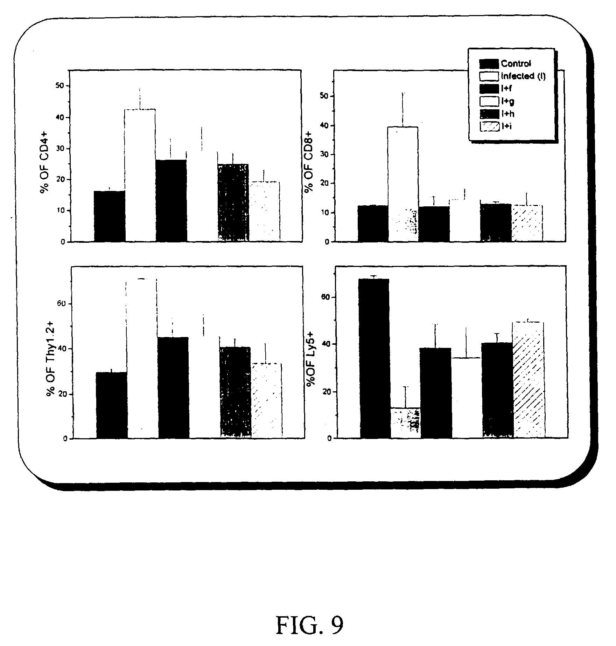

Having shown that the administration of Fludara and AZT is beneficial in LP-BM5 infected mice the outcome of the disease after suspension or continuation of different treatment regimes was evaluated next. As shown in Table X an increase in lymph node and spleen weight was observed in all treatment groups. The animals receiving AZT and Fludara sequentially are less affected than animals that received only Fludara or the simultaneous administration of both. This conclusion was confirmed by the other parameters investigated (Tables XI, XII, Fig.s 7-9). Only the phenotyping by flow citomecry of circulating lymphocytes are modified toward normalization in all experimental groups (Fig. 9).

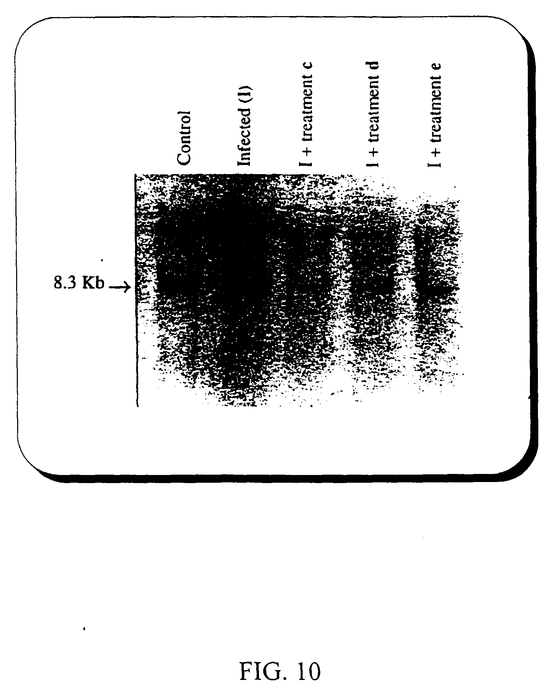

Finally, the long term toxicity of Fludara in MAIDS was evaluated. In fact, it is well known that nucleoside analogues that do not show a toxicity in short term cell cultures can cause a delayed toxicity by inhibiting the mitochondrial DNA polymerase. This effect has been described for ddC and FIAU. Thus, the mitochondrial DNA content in Fludara treated mice was evaluated and only treatment C causes a slight reduction of mitochondrial DNA content (20% vs. untreated mice) although this was not statistically significant. In Fig. 10, a representative experiment of mitochondrial DNA detection is shown. Mitochondrial DNA (mt DNA) content of control, infected, infected/treated mice at 10 weeks after infection is seen. From each sample 4 µg of total cellular DNA was digested with *Bam H*I, analysed on 0.8% agarose gel and detected with a ³²P-labeled 1544-bp probe. The relative amount of mt DNA was determined as described in experimental procedures. The different treatments are described in details in the legend of the table VII. All values are the mean ± SD of three mice. Only one representative sample per treatment is shown.

**Table I**

| Effect of Fludara on MAIDS. | | | |
|---|---|---|---|
| | Splenom. (% inh.) | Lymphad. (% inh.) | Hyperg. (% inh.) |
| Infected+ 0.065 mg/mouse | 0 | 20±15 | 12±10 |
| Infected+ 0.325 mg/mouse | 0 | 26±9 | 16±7 |
| Infected+ 0.650 mg/mouse | 0 | 49±6 | 19±4 |
| All values are mean ± S.D. of three animals. Absolute values for splenomegaly, lymphadenopathy and hypergammaglobulinemia in infected animals are reported in Table II. | | | |

**Table II**

| Inhibition of splenomegaly and lymphadenopathy of C57BL/6 mice infected with LP-BM5 by different concentrations of F-era-AMP. | | | |
|---|---|---|---|
| Mice | Body weight (g) | Spleen weight (g) | Lymph node w. (g) |
| Infected (I) | 25.90±1.00 | 1.22±0.37 | 3.22±0.45 |
| I+F-ara-AMP¹ | 23.74±1.18 | 1.01±0.24 | 2.63±0.57 |
| I+F-ara-AMP² | 23.35±1.15 | 0.87±0.20* | 2.11±0.54* |
| I+F-ara-AMP³ | 22.60±0.72 | 0.84±0.17* | 1.75±0.53* |

| | | | |
|---|---|---|---|
| (*)p<10⁻² (vs. Infected). C57BL/6 mice were infected by two successive intraperitoneal injections at 24 h interval each of 0.1 ml of the retroviral complex LP-BM5. After 7 weeks of infection mice were treated with F-ara-AMP as follows: | | | |
| ¹: 0.65 mg once a day 4 days/week for 3 weeks | | | |
| ²: 0.65 mg twice a day 5 days/week for 4 weeks | | | |
| ³: 3.00 mg once a day 5 days/week for 3 weeks Values are mean ± S.D. of 8 animals. | | | |

**Table III**

| Treatment scheme of Murine AIDS. | |
|---|---|
| Group | Fludara administration |
| a | 3 mg/day for 5 days for 4 weeks (i.p.) |
| b | 3 mg/twice a day for 5 days for 4 weeks (i.p.) |
| c | 10 mg/day in drinking water for 4 weeks |
| d | 15 mg/day in drinking water for 4 weeks |

**Table IV**

| Inhibition of splenomegaly and lymphadenopathy in C57BL/6 mice infected with LP-BM5 by F-ara-AMP. | | | |
|---|---|---|---|
| Mice | Body w.(g) | Spleen w.(g) | Lymph node w.(g) |
| Control | 20.24±2.12 | 0.10±0.03 | 0.03±0.01 |
| Infected (I) | 24.56±0.73 | 0.76±0.20 | 1.59±0.59 |
| I+F-ara-AMP^{a} | 21.90±1.45 | 0.50±0.25 | 0.54±0.32 |
| I+F-ara-AMP^{b} | 19.64±0.79 | 0.22±0.06* | 0.05±0.02* |
| I+F-ara-AMP^{C} | 20.57±0.35 | 0.31±0.09 | 0.16±0.16** |
| I+F-ara-AMP^{d} | 21.80±0.81 | 0.28±0.13** | 0.28±0.23** |

| | | | |
|---|---|---|---|
| (*)p<5x10⁻⁵; | | | |
| (**)p<5x10⁻³ ( vs. Infected). Mice, except control, were infected by means of two successive intraperitoneal injections of retroviral complex LP-BM5. After 7 weeks of infection, mice of group a were treated with intraperitoneal injections of 3.0 mg of F-ara-AMP once a day for 5 consecutive days; mice of group b were treated with intraperitoneal injections of 3.0 mg of F-ara-AMP twice a day for 5 consecutive days. Mice of group C were treated with 10 mg of F-ara-AMP in drinking water for 5 consecutive days; mice of group d were treated with 15 mg of F-ara-AMP in drinking water for 5 consecutive days. For each group the treatment lasted 4 weeks. Values are mean ± S.D. of 8 animals. | | | |

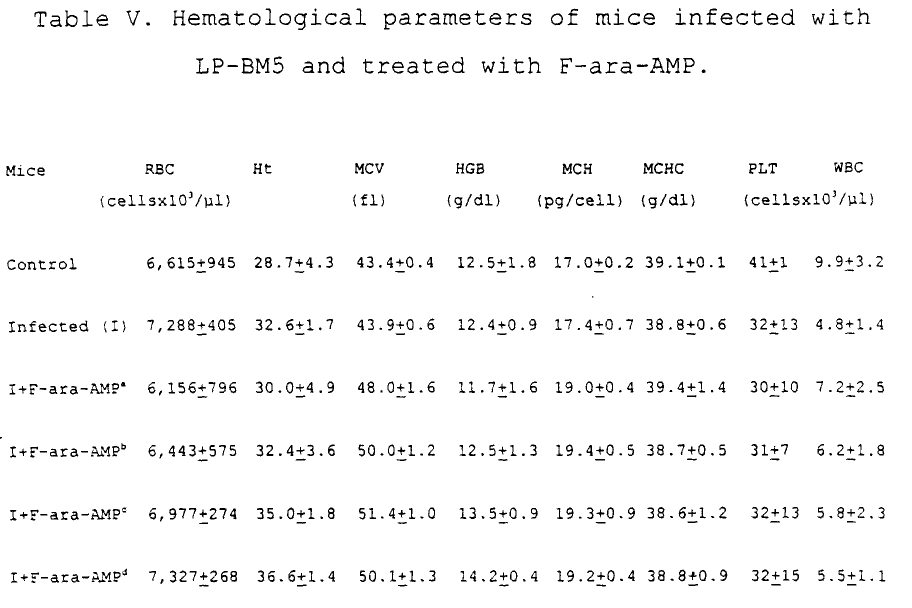

Mice, except control, were infected by means of two successive intraperitoneal injections of retroviral complex LP-BM5. After 7 weeks of infection, mice of group a were treated with intraperitoneal injections of 3.0 mg of F-ara-AMP once a day for 5 consecutive days; mice of group b were treated with intraperitoneal injections of 3.0 mg of F-ara-AMP twice a day for 5 consecutive days.

Mice of group c were treated with 10 mg of F-ara-AMP in drinking water for 5 consecutive days; mice of group d were treated with 15 mg of F-ara-AMP in drinking water for 5 consecutive days. For each group the treatment lasted 4 weeks.

**Table VI**

| Inhibition of BM5d proviral DNA in C57BL/6 mice infected with LP-BM5 by F-ara-AMP | | | | |
|---|---|---|---|---|
| | % of inhibition# | | | |
| | I+F-ara-AMP^{a} | I+F-ara-AMP^{b} | I+F-ara-AMP^{c} | I+F-ara-AMP^{d} |
| Spleen | 25.74±17.54 | 39.85±16.70* | 15.16±11.46 | 27.40±8.47* |
| Lymphnode | 19.76±11.10* | 54.63±13.12** | 30.33±19.67* | 12.16±5.48 |
| Bonemarrow | 0 | 22.94±7.40** | 28.05±8.84* | 21.69±9.02* |

| | | | | |
|---|---|---|---|---|
| (*)p<5x10⁻²; | | | | |
| (**)p<5x 10⁻³ (vs. Infected) | | | | |
| #The percentages of inhibition were referred to the infected (I) mice. Mice, except control, were infected by means of two successive intraperitoneal injections of retroviral complex LP-BM5. After 7 weeks of infection, mice of group a were treated with intraperitoneal injections of 3.0 mg of F-ara-AMP once a day for 5 consecutive days; mice of group b were treated with intraperitoneal injections of 3.0 mg of F-ara-AMP twice a day for 5 consecutive days. Mice of group c were treated with 10 mg of F-ara-AMP in drinking water for 5 consecutive days; mice of group d were treated with 15 mg of F-ara-AMP in drinking water for 5 consecutive days. For each group the treatment lasted 4 weeks. Values are mean ± S.D. of 8 animals. | | | | |

**Table VII**

| Inhibition of splenomegaly and lymphadenopathy of C57BL16 mice infected with LPBM5 by AZT and Fludarabine phosphate (F-ara-AMP) at 10 weeks after infection. | | | |
|---|---|---|---|
| Mice | Body w.(g) | Spleen w.(g) | Lymph node w.(g) |
| Control | 21.63±0.77 | 0.077±0.020 | 0.033±0.012 |
| Infected (I) | 25.47±1.49 | 1.130±0.430 | 2.910±1.050 |
| I+treatment a | 23.73±0.75 | 0.520±0.070** | 1.060±0.240** |
| I+treatment b | 22.00±0.65 | 0.580±0.150** | 0.550±0.330** |
| I+treatment c | 19.82±0.81 | 0.130±0.004* | 0.042±0.018* |
| I+treatment d | 19.37±0.32 | 0.110±0.018*^{△} | 0.020±0.001*^{△} |
| I+treatment e | 18.95±0.98 | 0.160±0.100* | 0.024±0.005* |
| Treatment a: mice were infected and treated with AZT in drinking water at the concentration of 250 mg/l for 6 weeks. Treatment b: mice were infected and treated with AZT in drinking water at the concentration of 250 mg/l for 10 weeks. Treatment c: after 6 weeks of infection, mice were treated with intraperitoneal injections of 3 mg of F-ara-AMP twice a day for 5 consecutive days and for 4 weeks. Treatment d: mice were infected and treated with AZT in drinking water at the concentration of 250 mg/l for 6 weeks. At this time point AZT treatment was interrupted and animals were treated with treatment c. Treatment e: mice were infected and treated with AZT alone in drinking water at the concentration of 250 mg/l for 6 weeks. After 6 weeks of AZT treatment animals were treated with the combination of AZT (250 mg/l) and F-ara-AMP at the concentration described above for further 4 weeks. Values are mean ± S.D. of 5 animals. | | | |

| | | | |
|---|---|---|---|
| (*)p<2x10⁻³ ; | | | |
| (**)p<5x10⁻² (vs. Infected) | | | |
| (△)p<5x10⁻² (vs. I+treatment b). Mice, except control, were infected by means of two successive intraperitoneal injections of retroviral complex LP-BM5. | | | |

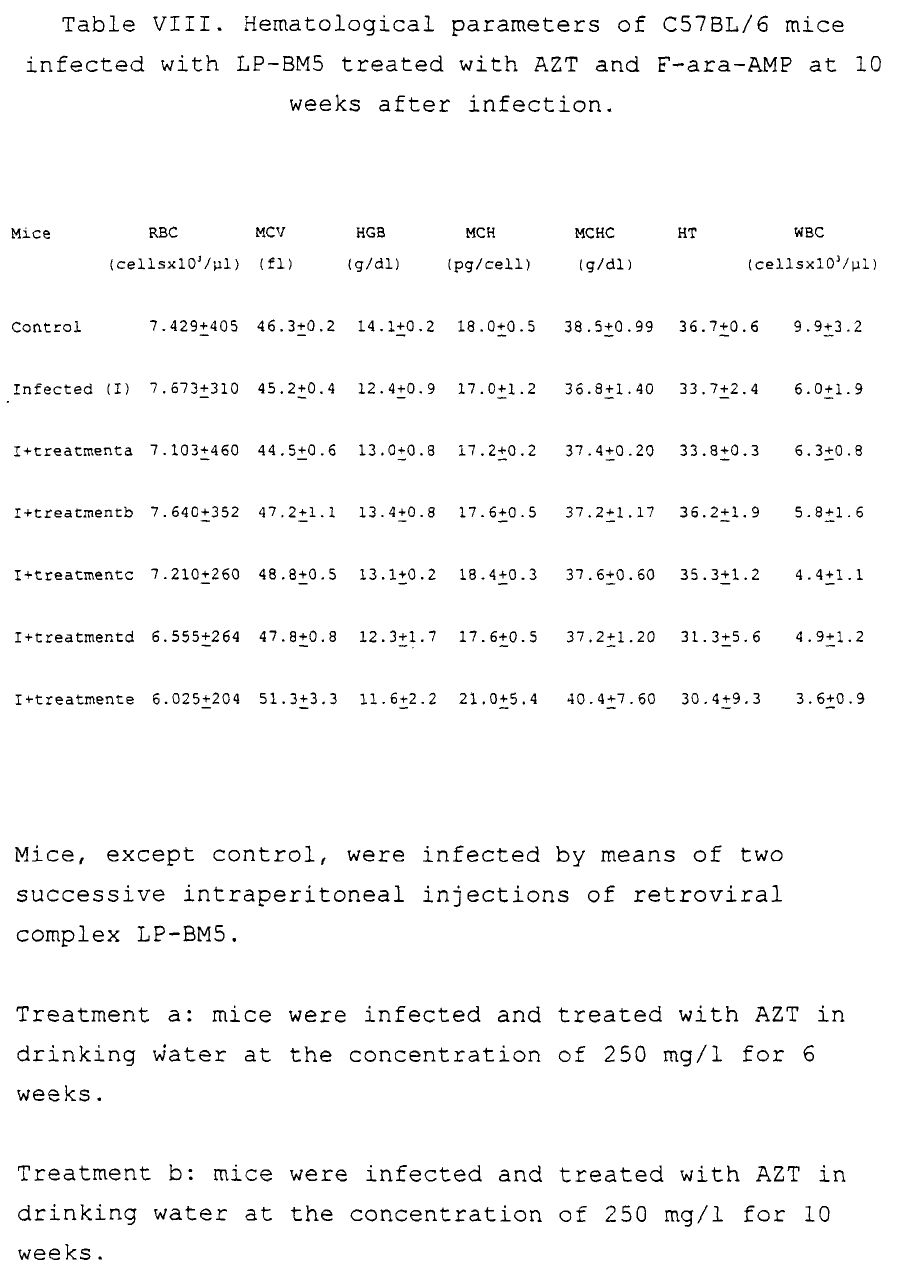

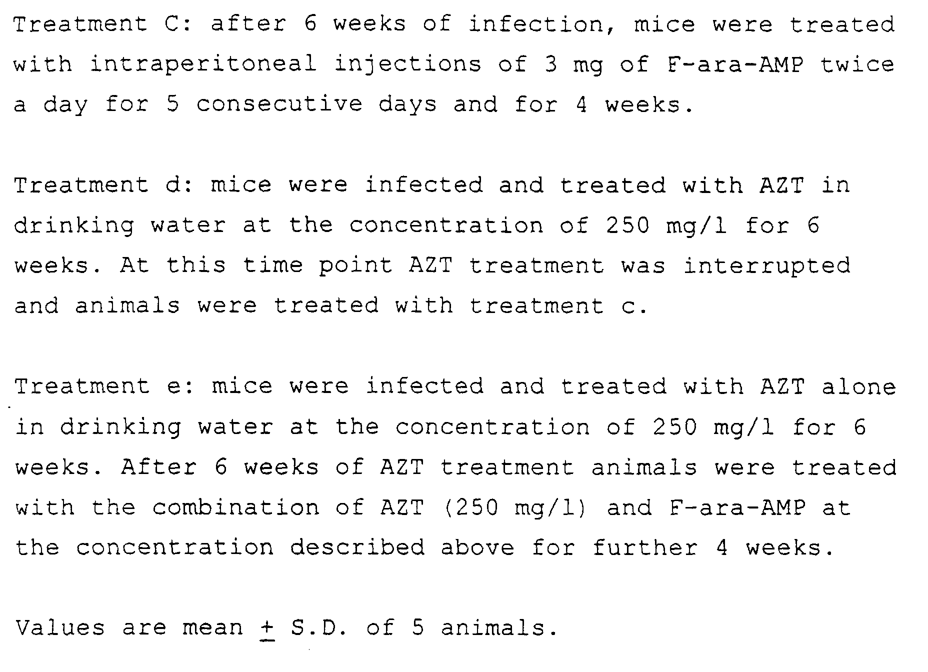

**Table IX**

| Copy numbers and percentages of inhibition of BM5d proviral DNA in C57BL/6 mice infected with LP-BM5 treated with AZT and F-ara-AM at 10 weeks after infection. | | | | | | |
|---|---|---|---|---|---|---|
| | Infected (I) | I+treatment a | I+treatment b | I+-treatment c | I+treatment d | I+treatment e |
| A | 147,305±19,305 | 135,000±20,000 | 39,669±19,026 | 37,900±15,717 | 8,614±2,027 | 31,350±12,23 |
| B | | 15.14±9.60 | 73.30±12.53** | 74.27+10.67** | 94.15±1.37***^{△} | 78.71±8.31** |
| C | 219,611±7,502 | 30,966±7,169 | 65,666±3 055 | 49,500±7758 | 6,842±1,342 | 28,193±11,674 |
| D | | 85.89±3.26** | 70.1O±1.39*** | 77.46±3.53** | 96.88±0.61***^{△} | 87.14±5.34** |
| E | 28,346±10,767 | 28,500±3,535 | 13,761±8,766 | 19,750±1,485 | 1,854±993 | 14,962±6,341 |
| F | | 4.135±5.847 | 51.51±30.83* | 30.32±5.24* | 93.45±3.51** | 47.22±22.37* |
| A, B: spleen, BM5d proviral DNA copy number #(A) and inhibition of BM5d (% vs. Infected) (B). C, D: lymph node, BM5d proviral DNA copy number #(C) and inhibition of BM5d (% vs. Infected) (D). E, F: bone marrow, BM5d proviral DNA copy number #(E) and inhibition of BM5d (% vs. Infected) (F). | | | | | | |

| | | | | | | |
|---|---|---|---|---|---|---|
| (*)p<5x10⁻²; | | | | | | |
| (**)p<5x10⁻⁴; | | | | | | |
| (***)p<5x10⁻⁷ (vs. infected); | | | | | | |
| (△)p<5x10⁻² (vs. I + treatment b, c, e). P values have been calculated on the percentage of inhibition of BM5d proviral DNA. | | | | | | |
| #Proviral DNA molecules per 0.3 µg of total cellular DNA, as assessed by cPCR. All values are the mean ± SD for five mice. | | | | | | |

The different treatments are described in details in the legend of the table VII.

**Table X**

| Inhibition of splenomegaly and lymph adenopathy of C57BL/6 mice infected with LP-BM5 by AZT and F-ara-AMP at 14 weeks after infection. | | | |
|---|---|---|---|
| Mice | Body w.(g) | Spleen w.(g) | Lymph node w.(g) |
| Control | 22.00±0.80 | 0.10±0.015 | 0.035±0.01 |
| Infected (1) | 28.77±0.13 | 1.23±0.200 | 5.300±1.14 |
| I+treatm. f | 21.00±1.40 | 0.35±0.060* | 0.220±0.06* |
| I+treatm. g | 20.90±1.22 | 0.41±0.200* | 0.290±0.05* |
| I+treatm. h | 22.25±0.35 | 0.36±0.005* | 0.210+0.01* |
| I+treatm. i | 21.35±1.05 | 0.37±0.105* | 0.400±0.23* |
| Treatment f: after 6 weeks of infection, mice were treated with intraperitoneal injections of 3 mg of F-ara-AMP twice a day for 5 consecutive days and for 4 weeks. At this time point F-ara-AMP treatment was interrupted and animals were treated with AZT in drinking water at the concentration of 250 mg/l for 4 weeks. Treatment g: after 6 weeks of infection, mice were treated with intraperitoneal injections of 3 mg of F-ara-AMP twice a day for 5 consecutive days and for 4 weeks. At this time point F-ara-AMP treatment was interrupted and animals were sacrificed at the same time as animals of treatment f. Treatment h: mice were infected and treated with AZT in drinking water at the concentration of 250 mg/l for 6 weeks. At this time point AZT treatment was interrupted and animals were treated with intraperitoneal injections of 3 mg of F-ara-AMP twice a day for 5 consecutive days and for 4 weeks. This treatment was interrupted and animals were sacrificed at the same time as animals of treatment f. Treatment i: mice were infected and treated with AZT alone in drinking water at the concentration of 250 mg/l for 6 weeks. After 6 weeks of AZT treatment animals were treated with the combination of AZT (250 mg/l) and F-ara-AMP at the concentration described above for further 4 weeks. At this time point these treatments were interrupted at the same time as animals of treatment f. Values are mean ± S.D. of 3 animals. | | | |

| | | | |
|---|---|---|---|
| (*)p<10⁻⁵ (vs. Infected). Mice, except control, were infected by means of two successive intraperitoneal injections of retroviral complex LP-BM5. | | | |

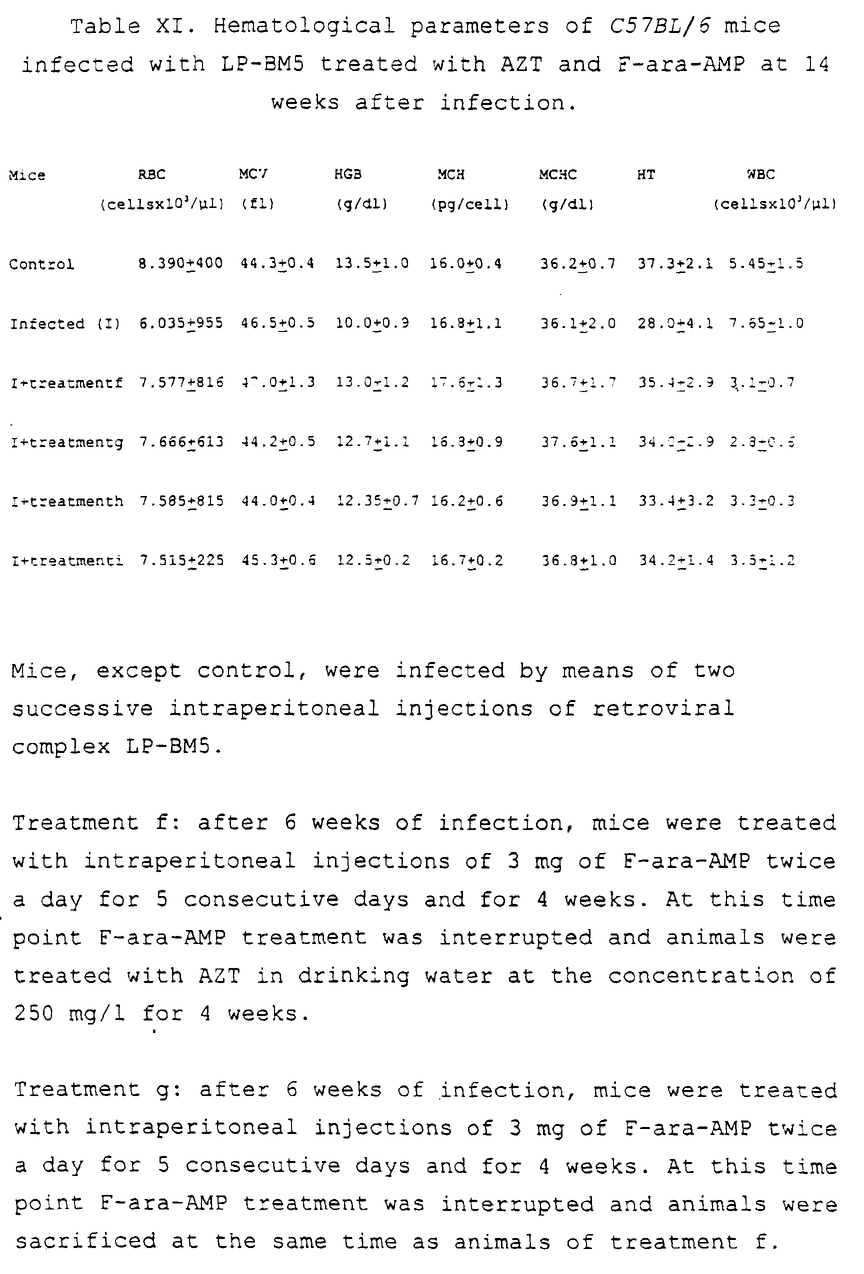

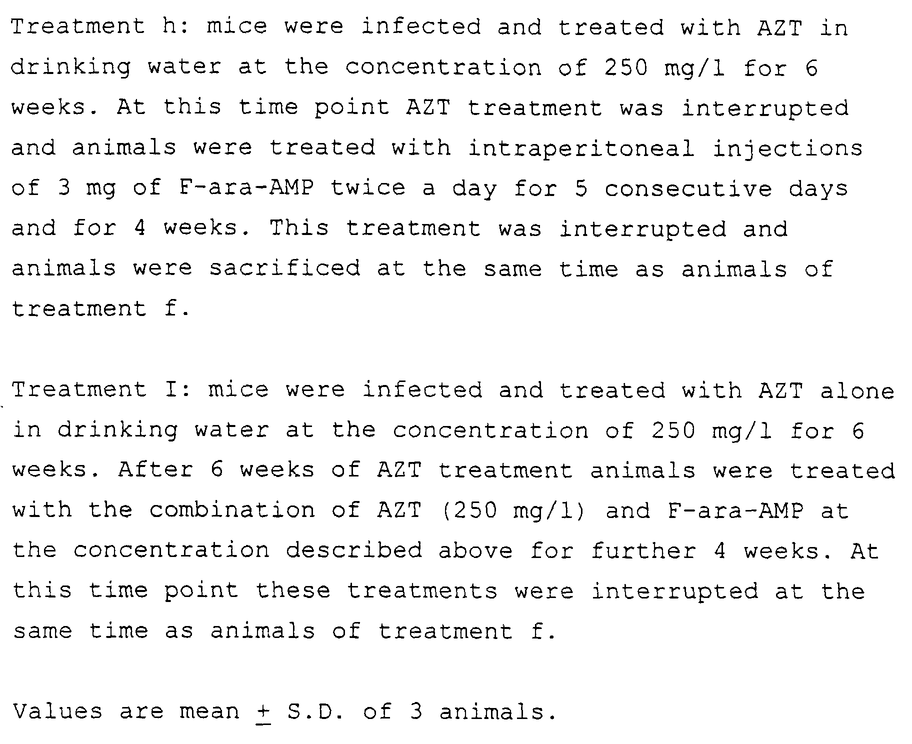

**Table XII**

| Copy numbers and percentages of inhibition of BM5d proviral DNA in C57BL/6 mice infected with LP-BM5 treated with AZT and F-ara-AMP at 15 weeks after infection. | | | | | |
|---|---|---|---|---|---|
| | Infected (I) | I+treatment f | I+treatment g | I+treatment h | I+treatment i |
| A | 197,000±74,953 | 35,600±2,066 | 135,000±47,444 | 58,000±2,828 | 73,400±10,748 |
| B | | 81.93±1.05***^{△} | 31.47±24.08 | 70.55±1.43*** | 62.74±5.46** |
| C | 272,750±98,750 | 42,100±11,230 | 180,000±8,544 | 27,500±3,535 | 40,500±12,020 |
| D | | 84.56±4.12*** | 34.00±3.13** | 89.91±1.29*** | 85.14±4.40** |
| E | 37,148±4,452 | 21,695±8,397 | 33,900±3,736 | 26,750±5,303 | 36,966±1,464 |
| F | | 47.63+32.86* | 7,17±6.2 | 27.98±14.27* | 1.56±2.71 |
| A, B: spleen, BM5d proviral DNA copy number #(A) and inhibition of BM5d (% vs. Infected) (B). C, D: lymph node, BM5d proviral DNA copy number #(C) and inhibition of BM5d (% vs. Infected) (D). E, F: bone marrow, BM5d proviral DNA copy number #(E) and inhibition of BM5d (% vs. Infected) (F). | | | | | |

| | | | | | |
|---|---|---|---|---|---|
| (*)p<5x10⁻²; | | | | | |
| (**)p<5x10⁻⁴; | | | | | |
| (***)p<5x10⁻⁶ (vs. Infected); | | | | | |
| (△)p<5x10-3 (vs. I + treatment h). P values have been calculated on the percentage of inhibition of BM5d proviral DNA. | | | | | |
| #Proviral DNA molecules per 0.3 µg of total cellular DNA, as assessed by cPCR. All values are the mean ± SD for three infected mice. | | | | | |

The different treatments are described in details in the legend of the table X.

## Claims

1. Pharmaceutical composition or composition package containing a nucleoside analogue (a) selected from the group consisting of reverse transcriptase inhibitors being pyrimidine nucleoside analogues, characterized in that it further contains a nucleoside analogue (b) selected from the group consisting of arabinonucleosides and halogenated and/or 5*'* phosphorylated and/or 2*'* and/or 3*'* desoxy- derivatives thereof.

2. Pharmaceutical composition or composition package according to claim 1, characterized in that the nucleoside analogue (a) is 3*'*-Azido-3*'*-desoxythymidin.

3. Pharmaceutical composition or composition package according to claim 1 or 2, characterized in that the nucleoside analogue (b) is an, optionally 2*'* and/or 3*'* desoxy-, adeninarabinoside halogenated at position 2 and/or phosphorylated at position 5*'*.

4. Pharmaceutical composition or composition package according claim 3, characterized in that the nucleoside analogue (b) is 2-X-9-β-D-Arabinofuranosyl-9H- purin-6-amine or the 5*'* mono-, di-, or triphosphate thereof, whereby X is F, Cl or Br, preferably F.

5. Use of a nucleoside analogue (a) selected from the group consisting of reverse transcriptase inhibitors being pyrimidine nucleoside analogues and of a nucleoside analogue (b) selected from the group consisting of arabinonucleosides and halogenated and/or phosphorylated and/or 2*'* and/or 3*'* desoxyderivatives thereof for manufacturing a pharmaceutical composition or composition package for treating diseases caused by lentivirus infection, in particular for treating AIDS.

6. Use according to claim 5, whereby the nucleoside analogue (a) is 3*'*-Azido-3*'*-desoxythymidin.

7. Use according to claim 5 or 6, whereby the nucleoside analogue (b) is an, optionally 2*'* and/or 3*'* desoxy-, adeninarabinoside halogenated at position 2 and/or phosphorylated at position 5*'*.

8. Use according to claim 7, whereby the nucleoside analogue (b) is 2-X-9-β-D-Arabinofuranosyl-9H-purin-6-amine or the 5*'* mono-, di-, or triphosphate thereof and whereby X is F, Cl or Br, preferably F.

9. Use according to one of the claims 5 to 8 in the embodiment of a pharmaceutical composition package comprising a package component (A) containing the nucleoside analogue (a) and a package component (B) containing the nucleoside analogue (b), whereby the package components (A) and (B) are determined to be administered according to a therapy plan comprising sequential administration of the package components (A) and (B).

10. Use according to claim 9, whereby the package component (A) is determined for administration prior to the administration of the package component (B).

11. Use according to claim 10, whereby the package component (A) is determined for administration for a period of time of 2 - 20 weeks and the package component (B) is determined for administration for a subsequent period of time of 2 - 20 weeks, whereby the administration of the of the package component (A) is either maintained or interrupted during the administration of the package component (B).

12. Use according to one of the claims 9 to 11, whereby the package components (A) and (B) are determined for repeating cycles of administration.

13. Process for making a pharmaceutical composition or a pharmaceutical composition package, characterized in that a nucleoside analogue (a) selected from the group consisting of reverse transcriptase inhibitors being pyrimidine nucleoside analogues and a nucleoside analogue (b) selected from the group consisting of arabinonucleosides and halogenated and/or phosphorylated and/or 2*'* and/or 3*'* desoxy- derivatives thereof are used, whereby optionally usual galenic additives are admixed to the a mixture of the nucleoside analogues (a) and (b) or are separately admixed to package components (A) and (B), each containing one of the nucleoside analogues (a) and (b), respectively.

14. Process according to claim 13, characterized in that the nucleoside analogue (a) is 3*'*-Azido-3*'*-desoxythymidin.

15. Process according to claim 13 or 14, characterized in that the nucleoside analogue (b) is an, optionally 2*'* and/or 3*'* desoxy-, adeninarabinoside halogenated at position 2 and/or phosphorylated at position 5*'*.

16. Process according to claim 15, characterized in that the nucleoside analogue (b) is 2-X-9-β-D-Arabinofuranosyl-9H-purin-6-amine or the 5*'* mono-, di-, or triphosphate thereof, whereby X is F, Cl or Br, preferably F.

17. Method for treating diseases caused by lentivirus infections, in particular for treating AIDS, whereby a pharmaceutical composition or composition package according to one of the claims 1 to 4 is administered to the infected organism.

18. Method according to claim 17, whereby a composition package comprising a package component (A) containing the nucleoside analogue (a) and a package component (B) containing the nucleoside analogue (b) is used, and whereby the package components (A) and (B) are administered according to a therapy plan consisting of sequential administration of the package components (A) and (B).

19. Method according to claim 18, whereby the package component (A) is administered prior to the administration of the package component (B).

20. Method according to claim 19, whereby the package component (A) is administered for a period of time of 2 - 20 weeks and the package component (B) is administered for a subsequent period of time of 2 - 20 weeks, whereby the administration of the of the package component (A) is either maintained or interrupted during the administration of the package component (B).

21. Method according to one of the claims 18 to 20, whereby the package components (A) and (B) are administered in repeating cycles.
